(19) Europäisches Patentamt / European Patent Office / Office européen des brevets

(11) **EP 3 967 218 B1**

(12) **EUROPEAN PATENT SPECIFICATION**

(45) Date of publication and mention of the grant of the patent:
**16.10.2024 Bulletin 2024/42**

(21) Application number: **21196224.6**

(22) Date of filing: **13.09.2021**

(51) International Patent Classification (IPC):
*A61B 5/00* *(2006.01)*   *A61B 5/367* *(2021.01)*
*A61B 5/0538* *(2021.01)*   *A61B 5/283* *(2021.01)*
*A61B 5/287* *(2021.01)*   *A61B 5/339* *(2021.01)*
*A61B 18/12* *(2006.01)*   *A61B 18/14* *(2006.01)*
*A61B 5/349* *(2021.01)*   *G16H 50/20* *(2018.01)*
*G16H 40/67* *(2018.01)*

(52) Cooperative Patent Classification (CPC):
**A61B 5/7267; A61B 5/287; A61B 5/339;**
**A61B 5/349; A61B 5/367; A61B 18/12;**
**G16H 40/67; G16H 50/20;** A61B 18/1492

(54) **LOCAL ACTIVATION TIME ANALYSIS SYSTEM**

LOKALES AKTIVIERUNGSZEITANALYSESYSTEM

SYSTÈME D'ANALYSE DE TEMPS D'ACTIVATION LOCALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.09.2020 US 202063077780 P**
**21.06.2021 US 202117352771**

(43) Date of publication of application:
**16.03.2022 Bulletin 2022/11**

(73) Proprietor: **Biosense Webster (Israel) Ltd Yokneam, 2066717 (IL)**

(72) Inventor: **GLINER, Vadim**
**2066717 Yokneam (IL)**

(74) Representative: **Carpmaels & Ransford LLP**
**One Southampton Row**
**London WC1B 5HA (GB)**

(56) References cited:
**CN-A- 108 836 302**   **US-A1- 2018 296 108**

• **LOZOYA ROCIO CABRERA ET AL: "Model-Based Feature Augmentation for Cardiac Ablation Target Learning From Images", IEEE TRANSACTIONS ON BIOMEDICAL ENGINEERING, IEEE, USA, vol. 66, no. 1, 1 January 2019 (2019-01-01), pages 30 - 40, XP011700871, ISSN: 0018-9294, [retrieved on 20181218], DOI: 10.1109/TBME.2018.2818300**
• **ZHENGE JIA ET AL: "Personalized Deep Learning for Ventricular Arrhythmias Detection on Medical IoT Systems", ARXIV.ORG, CORNELL UNIVERSITY LIBRARY, 201 OLIN LIBRARY CORNELL UNIVERSITY ITHACA, NY 14853, 18 August 2020 (2020-08-18), XP081743536**

**Description**

FIELD OF THE INVENTION

The present invention relates systems to find local activation times of intracardiac electrogram (IEGM) signals.

BACKGROUND

Electrode catheters have been in common use in medical practice for many years. They are used to stimulate and map electrical activity in the heart and to ablate sites of aberrant electrical activity. In use, the electrode catheter is inserted into a major vein or artery, e.g., femoral vein, and then guided into the chamber of the heart of concern. A typical ablation procedure involves the insertion of a catheter having a one or more electrodes at its distal end into a heart chamber. A reference electrode may be provided, generally taped to the skin of the patient or by means of a second catheter that is positioned in or near the heart. RF (radio frequency) current is applied between the catheter electrode(s) of the ablating catheter and an indifferent electrode (which may be one of the catheter electrodes), and current flows through the media between the electrodes, i.e., blood and tissue. The distribution of current may depend on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive. In some applications, irreversible electroporation may be performed to ablate the tissue.

Electrophysiological (EP) cardiac mapping is a diagnostic medical procedure for identifying locations of cardiac dysfunction within a heart. Time-varying electrocardiogram (ECG) signals are received by electrodes contacting points along the surface of a patient's heart. The signals are processed and different metrics regarding cardiac functions are computed from the processed (ECG) signals, which are then spatially mapped onto an image of the heart. The map is then outputted for a medical professional to analyze.

The analysis of cardiac signals sometimes involves synchronizing to the timing of the ECG signals. For example, U.S. Patent Application Publication No. 2013/0123652 describes a method for analyzing signals, including sensing a time-varying intra-cardiac potential signal and finding a fit of the time-varying intra-cardiac potential signal to a predefined oscillating waveform. The method further includes estimating an annotation time of the signal responsive to the fit.

Electrophysiological (EP) cardiac mapping, or cardiac electro-anatomical mapping, is used to identify regions in the heart tissue that are dysfunctional. An intra-body probe, typically a catheter with multiple mapping electrodes disposed along the body of the catheter near the catheter distal end, is inserted into a cavity of the heart. Time varying electro-cardiogram (ECG) signals are recorded at multiple contact points between the mapping electrodes and the heart tissue. The multiple ECG electrodes are then moved to different contact positions with the heart tissue and the process is repeated. Then, metrics regarding cardiac function are computed from the local ECG signals, which are mapped spatially across the surface of the heart cavity. The mapping assists the medical professional to identify regions of heart dysfunction.

Electrical sources in the heart, such as the sinoatrial (SA) and atrioventricular (AV) nodes initiate electrical activity waves that propagate over the heart triggering the muscle tissue in the atria and ventricles to contract in a characteristic sinus rhythm. When the activity wave-front reaches the multiple mapping electrodes during each cardiac cycle, the characteristic ECG waveforms are detected at the multiple mapping electrodes. These waveforms are time-shifted due to the different arrival times of the same wave-front at the different multiple electrodes contacting the tissue at different spatial locations along the surface of the heart cavity.

The arrival times of the ECG waveforms detected at the multiple mapping electrodes can be used to map the propagation time and/or velocity of the activity wave across the heart. The mapping of the activity wave is performed with respect to a single time reference indicative of the cardiac cycle known herein as the reference annotation time.

The reference annotation time can be computed by processing the ECG signals obtained from a body surface (BS) electrode, or from an intra-cardiac (IC) reference electrode on an additional catheter and placed in contact with the surface of the cardiac chamber. Typically, the physician designates whether the reference annotation time is computed from a BS or IC channel, depending on the suspected pathology.

US2018/0296108A1 discusses systems and methods for facilitating processing of cardiac information based on sensed electrical signals that include a processing unit configured to receive a set of electrical signals; receive an indication of a measurement location corresponding to each electrical signal of the set of electrical signals; and generate, based on at least one of an annotation waveform corresponding to each electrical signal of the set of electrical signals and a set of annotation mapping values, an annotation histogram.

SUMMARY

There is provided in accordance with an embodiment of the present invention, a system to find local activation times of intracardiac electrogram (IEGM) signals according to independent claim 1.

Alternative embodiments are defined in dependent claims 2-9.

Further in accordance with another embodiment of the present invention it is provided a software product according to independent claim 10.

BRIEF DESCRIPTION OF THE DRAWINGS

The present invention will be understood from the following detailed description, taken in conjunction with the drawings in which:

Fig. 1 is a pictorial illustration of a system for performing catheterization procedures on a heart, constructed and operative in accordance with an exemplary embodiment;

Fig. 2 is a flowchart including steps in a method of operation of an electrophysiological laboratory subsystem in the system of Fig. 1;

Fig. 3 is a view of displayed annotated intracardiac electrogram signals rendered using the system of Fig. 1;

Fig. 4 is a block diagram view of the system of Fig. 1;

Fig. 5 is a flowchart including steps in a method of operation of a remote server in the system of Fig. 1;

Fig. 6 is a schematic view of an artificial neural network for use with the system of Fig. 1;

Fig. 7 is a flowchart including sub-steps in a step of the method of Fig. 5;

Fig. 8 is a flowchart including steps in a method to process an intracardiac electrogram signal using the trained artificial neural network of Fig. 6; and

Fig. 9 is a schematic view of a displayed electroanatomic map rendered by the system of Fig. 1.

DESCRIPTION OF EXAMPLE EMBODIMENTS

OVERVIEW

One of the main challenges in electrophysiology (EP) is finding an accurate intracardiac electrogram (IEGM) signal annotation algorithm to select correct local activation times (LATs) from the IEGM signals, for example, to generate a LAT map. One method to automatically find the LATs includes finding the maximum negative slope of the signal within each window of interest (WOI) and assign the LAT to that point. The WOI in typically set by the system based on detecting the QRS complex of a signal captured by one or more body surface electrodes. Detecting the maximum negative slope does not provide a broad solution to the above problem as in some cases there may be many such slopes in the WOI and the wrong slope may be selected by the algorithm. Therefore, the physician has the option to manually change the computed LAT to a different point in the WOI.

Embodiments of the present invention solve the above problems by training an artificial neural network (ANN) using deep learning techniques to find LATs for corresponding IEGM signals. The ANN is trained using IEGM signals and corresponding LAT annotations manually annotated by annotation personnel (e.g., physicians or other medical professionals) provided by different EP laboratories (labs). The manual annotations may be correcting an automatically computed annotation or may be providing an initial annotation for an IEGM signal.

In some embodiments, during the training of the ANN, different LAT annotations are not provided an equal weight. For example, a loss function used to train the ANN may include weights, which may be associated with each respective IEGM signal and LAT annotation pair in order to weight the contribution of each pair in the training. In some embodiments, a binary cross-entropy (BCE) loss function may be used.

In some embodiments, the weight assigned to an IEGM signal and LAT annotation pair (e.g., for use in the loss function) may be computed responsibly to the LAT annotation experience level of the annotation person who manually determined that LAT annotation. In this way, the ANN may be trained by giving more weight to more experienced annotation personnel.

The LAT annotation experience levels of different annotation personnel may be estimated by searching a database including scientific literature publications to find the number of search matches (e.g. the number of scientific literature publications) for the different annotation personal in the realm of LAT annotation. For example, searching for "John Smith and LAT annotation" may provide 50 matches, while searching for "Tim Jones and LAT annotation" may provide 400 matches. In such a case, LAT annotations provided by Tim Jones are provided much more weight in the training than LAT annotations provided by John Smith. Any suitable database may be searched, for example, Google Scholar, Scopus or Web of Science.

SYSTEM DESCRIPTION

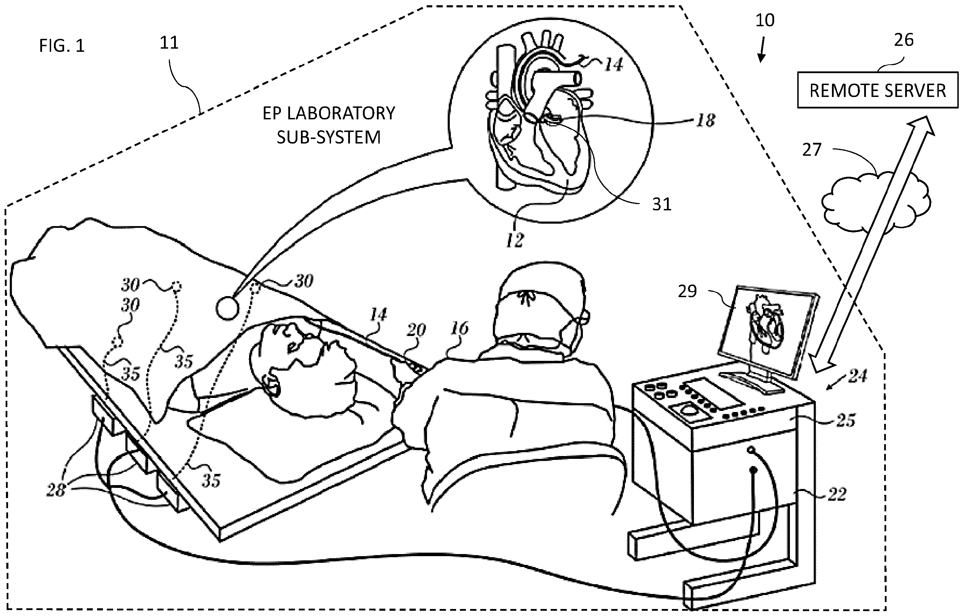

Reference is now made to Fig. 1, which is a pictorial illustration of a medical system 10 for performing catheterization procedures on a heart 12, constructed and operative in accordance with an embodiment of the present disclosure.

The medical system 10 may be configured to evaluate electrical activity and perform ablative procedures on the heart 12 of a living subject. The system 10 comprises EP laboratory sub-systems 11 (only one shown for the sake of simplicity), which capture EP data. The medical system 10 also comprises a remote server 26, e.g., a cloud computing device, to which EP data is sent for storage and/or processing by the EP laboratory sub-systems 11 over a network 27. The EP data may be compressed in the EP laboratory sub-systems 11 and sent to the remote server 26 over the network 27 in compressed form.

One of the EP laboratory sub-systems 11 is now described in more detail below by way of example only. The different EP laboratory sub-systems 11 may comprises the same or different EP lab equipment to provide EP data to the remote server 26 for processing. The EP laboratory sub-system 11 of Fig. 1 comprises a catheter 14, which is percutaneously inserted by an operator 16 through the patient's vascular system into a chamber or vascular structure of the heart 12. The operator 16, who is typically a physician, brings the catheter's distal end 18 into contact with the heart wall, for example, at an ablation target site. Electrical activation maps may be prepared, according to the methods disclosed in U.S. Patent Nos. 6,226,542, 6,301,496, and 6,892,091. One commercial product embodying elements of the system 10 is available as the CARTO® 3 System, available from Biosense Webster, Inc., Irvine, CA. This system may be modified by those skilled in the art to embody the principles described herein

Areas determined to be abnormal, for example by evaluation of the electrical activation maps, can be ablated by application of thermal energy, e.g., by passage of radiofrequency electrical current through wires in the catheter to one or more electrodes at the distal end 18, which apply the radiofrequency energy to the myocardium. The energy is absorbed in the tissue, heating it to a point at which it permanently loses its electrical excitability. When successful, this procedure creates non-conducting lesions in the cardiac tissue, which disrupt the abnormal electrical pathway causing the arrhythmia. The principles of the disclosure can be applied to different heart chambers to diagnose and treat many different cardiac arrhythmias.

The catheter 14 typically comprises a handle 20, having suitable controls on the handle to enable the operator 16 to steer, position and orient the distal end 18 of the catheter 14 as desired for the ablation. To aid the operator 16, a distal portion of the catheter 14 contains position sensors (not shown) that provide signals to processing circuitry 22, located in a console 24. The processing circuitry 22 may fulfill several processing functions as described below.

Wire connections 35 may link the console 24 with body surface electrodes 30 and other components of a positioning sub-system for measuring location and orientation coordinates of the catheter 14. The processing circuitry 22 or another processor (not shown) may be an element of the positioning subsystem. Catheter electrodes 31 and the body surface electrodes 30 may be used to measure tissue impedance at the ablation site as taught in U.S. Patent No. 7,536,218. Temperature sensors (not shown), typically a thermocouple or thermistor, may be mounted on ablation surfaces on the distal portion of the catheter 14 as described below.

The console 24 typically contains one or more ablation power generators 25. The catheter 14 may be adapted to conduct ablative energy to the heart using any known ablation technique, e.g., radiofrequency energy, ultra-sound energy, irreversible electroporation and laser-produced light energy. Such methods are disclosed in U.S. Patent Nos. 6,814,733, 6,997,924, and 7,156,816.

In one embodiment, the positioning subsystem comprises a magnetic position tracking arrangement that determines the position and orientation of the catheter 14 by generating magnetic fields in a predefined working volume and sensing these fields at the catheter, using field generating coils 28. The positioning subsystem is described in U.S. Patent Nos. 7,756,576, and 7,536,218.

As noted above, the catheter 14 is coupled to the console 24, which enables the operator 16 to observe and regulate the functions of the catheter 14. Console 24 includes the processing circuitry 22, generally a computer with appropriate signal processing circuits. The processing circuitry 22 is coupled to drive a display 29 (e.g., a monitor). The signal processing circuits typically receive, amplify, filter and digitize signals from the catheter 14, including signals generated by sensors such as electrical, temperature and contact force sensors, and location sensing electrodes 31 located distally in the catheter 14. The digitized signals are received and used by the console 24 and the positioning system to compute the position and orientation of the catheter 14, and to analyze the electrical signals from the electrodes. In some embodiments, the digitized signals are sent (and optionally compressed prior to sending) to the remote server 26 to compute the position and orientation data, and/or to analyze the electrical signals from the electrodes 30, 31, and/or to use the electrical signals and associated data to train an artificial neural network, described in more detail below.

In order to generate electroanatomic maps, the processing circuitry 22 typically comprises a mapping module including an electroanatomic map generator, an image registration program, an image or data analysis program and a graphical user interface configured to present graphical information on the display 29. In some embodiments, some or all of the functionality of the mapping module is performed by the remote server 26.

Typically, the system 10 includes other elements, which are not shown in the figures for the sake of simplicity. For example, the system 10 may include an electrocardiogram (ECG) monitor, coupled to receive signals from one or more of the body surface electrodes 30, in order to provide an ECG synchronization signal to the console 24 or the remote server 26. In some embodiments, some or all of the functionality of the ECG monitor is performed by the remote server 26. As mentioned above, the system 10 typically also includes a reference position sensor, either on an externally-applied reference patch attached to the exterior of the subject's body, or on an internally-placed catheter, which is inserted into the heart 12 maintained in a fixed position relative to the heart 12. Conventional pumps and lines for circulating liquids through the catheter 14 for cooling the ablation site may be provided. The system 10 and/or the remote server 26 may receive image data from an external imaging modality, such as an MRI unit or the like and includes image processors that can be incorporated in or invoked (e.g., by the processing circuitry 22 and/or the remote server 26) for generating and displaying images.

In practice, some or all of the functions of the processing circuitry 22 may be combined in a single physical component or, alternatively, implemented using multiple physical components. These physical components may comprise hard-wired or programmable devices, or a combination of the two. In some embodiments, at least some of the functions of the processing circuitry 22 may be carried out by a programmable processor under the control of suitable software. This software may be downloaded to a device in electronic form, over a network, for example. Alternatively, or additionally, the software may be stored in tangible, non-transitory computer-readable storage media, such as optical, magnetic, or electronic memory.

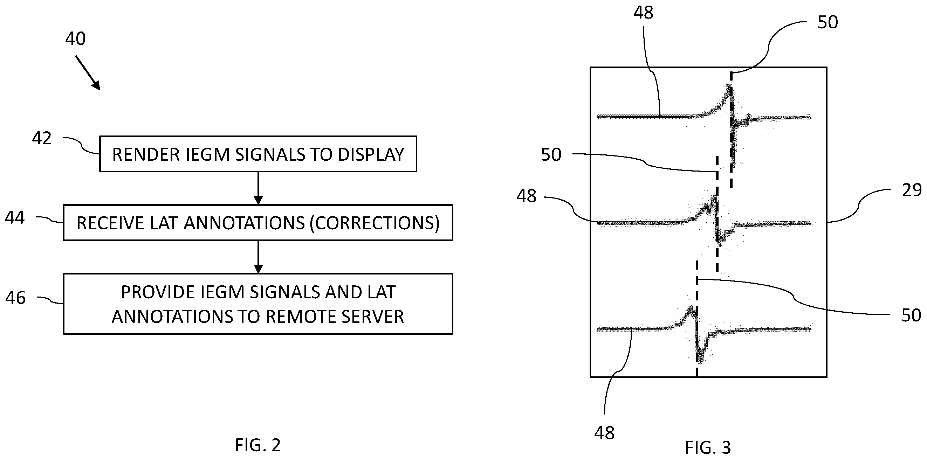

Reference is now made to Figs. 2 and 3. Fig. 2 is a flowchart 40 including steps in a method of operation of one of the electrophysiological laboratory sub-systems 11 in the system 10 of Fig. 1. Fig. 3 is a view of displayed annotated IEGM signals 48 rendered using the system 10 of Fig. 1.

Each EP laboratory sub-system 11 includes the catheter 14 configured to be inserted into at least one cardiac chamber of at least one living subject, and to capture respective IEGM signals 48 from the at least one cardiac chamber. Any suitable type of catheter or catheters 14 may be used in each EP laboratory sub-systems 11. The processing circuitry 22 is configured to receive the IEGM signals 48 and render (block 42) representations of the IEGM signals 48 to the display 29 and receive (block 44) local activation time (LAT) annotations 50 of the corresponding IEGM signals 48 manually annotated by an annotation person of that EP laboratory sub-system 11. The IEGM signals 48 may be first automatically annotated by an algorithm running on the processing circuitry 22. The annotation person may then correct the automated annotation with a manual annotation by marking the local activation time annotations 50 on the displayed IEGM signals 48. In some embodiments, the annotation person may view the displayed IEGM signals 48 on the display 29 (without the processing circuitry 22 computing an automated LAT annotation) and then mark the local activation time annotations 50 on the displayed IEGM signals 48.

The processing circuitry 22 of the EP laboratory sub-system 11 is configured to provide (block 46) the IEGM signals 48 and the corresponding local activation time annotations 50 to the remote server 26. In some embodiments, the EP laboratory sub-systems 11 are configured to provide the IEGM signals 48 and corresponding local activation time annotations 50 to the remote server 26 over the network 27 (Fig. 1) via suitable network interfaces.

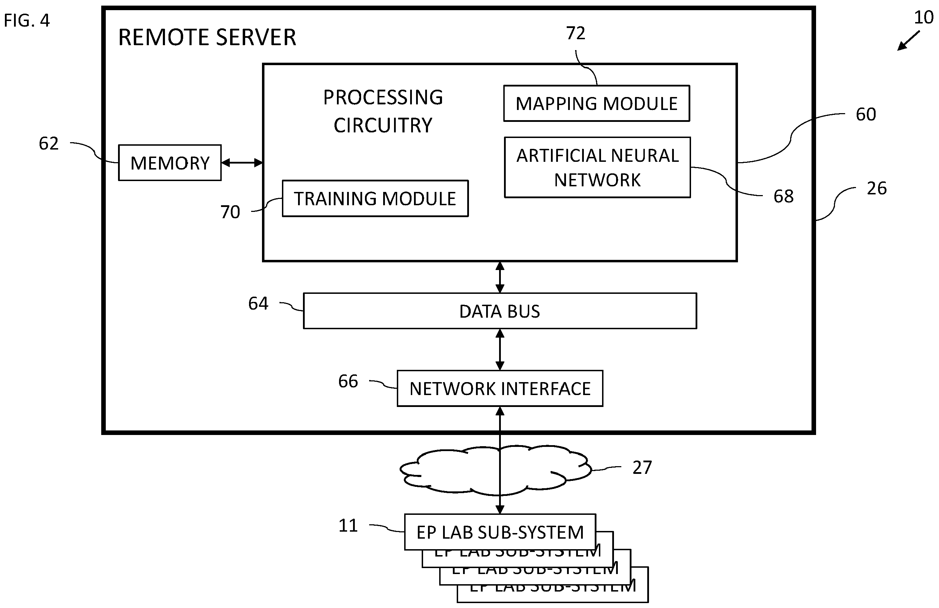

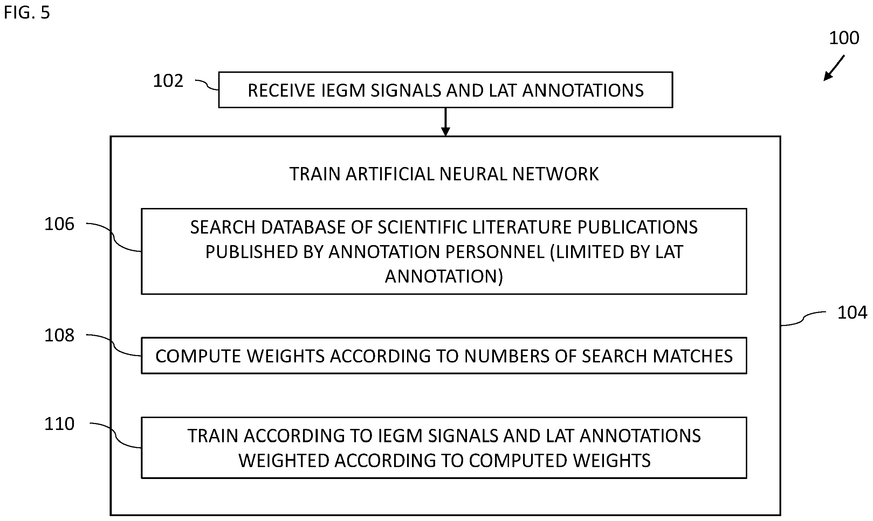

Reference is now made to Figs. 4 and 5. Fig. 4 is a block diagram view of the system 10 of Fig. 1. Fig. 5 is a flowchart 100 including steps in a method of operation of the remote server 26 in the system 10 of Fig. 1.

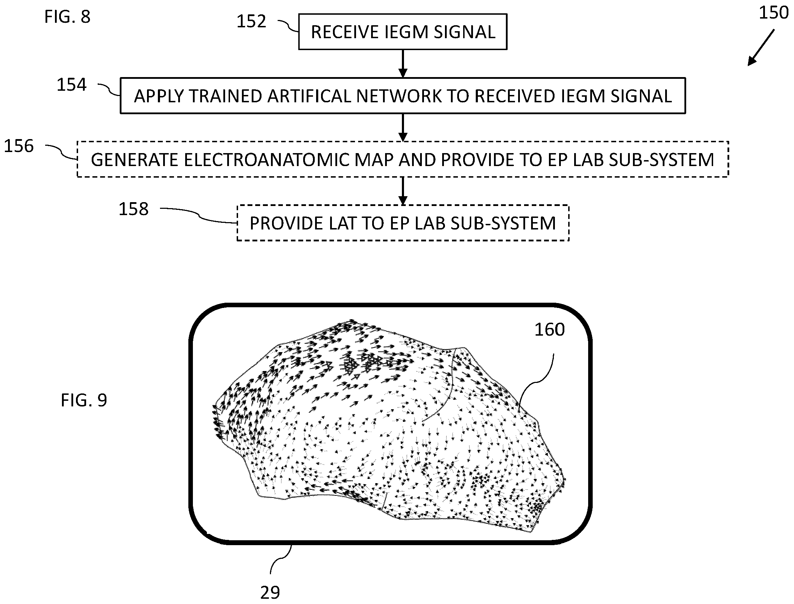

The remote server 26 includes processing circuitry 60, a memory 62, a data bus 64, and a network interface 66. The processing circuitry 60 is configured to run software to perform various signal processing and computation tasks, including an artificial neural network 68, a training module 70, and a mapping module 72. The training module 70 is configured to train the artificial neural network 68 as described in more detail below with reference to Figs. 5-7. The mapping module 72 is configured to generate EP maps responsively to cardiac signals and other data captured from a living subject as described in more detail with reference to Figs. 8 and 9.

In practice, some or all of the functions of the processing circuitry 60 may be combined in a single physical component or, alternatively, implemented using multiple physical components. These physical components may comprise hard-wired or programmable devices, or a combination of the two. In some embodiments, at least some of the functions of the processing circuitry 60 may be carried out by a programmable processor under the control of suitable software. This software may be downloaded to a device in electronic form, over a network, for example. Alternatively, or additionally, the software may be stored in tangible, non-transitory computer-readable storage media, such as optical, magnetic, or electronic memory.

The memory 62 is configured to store data used by the processing circuitry 60. The data bus 64 is configured to transfer data between the various elements of the remote server 26 for example, between the processing circuitry 60 and the network interface 66.

The training module 70 running on the processing circuitry 60 is configured to receive (block 102), from the EP laboratory sub-systems 11, the IEGM signals 48 (captured in the respective EP laboratory sub-systems 11) and corresponding local activation time annotations 50 of the IEGM signals 48 manually annotated by respective annotation personnel. In other words, the training module 70 is configured to receive IEGM signals 48 and corresponding local activation time annotations 50 manually annotated by one annotation person of one of the EP laboratory sub-systems 11, and other IEGM signals 48 and corresponding local activation time annotations 50 manually annotated by another annotation person of another one of the EP laboratory sub-systems 11, and so on.

The training module 70 running on the processing circuitry 60 is configured to train (block 104) the artificial neural network 68 to find local activation times of IEGM signals responsively to training data including the IEGM signals 48 and the corresponding local activation time annotations 50. The step of block 104 includes substeps of blocks 106-110 described in more detail below.

The training module 70 running on the processing circuitry 60 is configured to search (block 106) a database of scientific literature publications (e.g., Google Scholar, Scopus, Web of Science) responsively to the respective annotation personnel (who supplied the local activation time annotations 50) as search strings, yielding respective numbers of search matches indicative of the local activation time annotation experience level of the respective annotation personnel. In other words, the database is searched with different search strings for each of the annotation personnel (e.g., "J. Smith", "T. Jones", etc.) to yield a number of search matches (e.g., 5 matches for J. Smith and 40 matches for T. Jones, etc.) for each of the annotation personnel. In some cases, the search for a given annotation person may yield no search matches. The search may be performed using any suitable software script, e.g., using a web crawler such as pybliometrics 2.5.0 to access Scopus. The respective numbers of search matches may be respective numbers of the scientific literature publications matching the respective annotation personnel e.g., 5 publications for J. Smith and 40 publications for T. Jones, etc.).

In some embodiments, the training module 70 running on the processing circuitry 60 is configured to limit searching of the database to scientific literature publications describing local activation time annotation and/or IEGM and/or electrocardiogram annotation and/or EP annotation etc. Limiting the searching to one or more of the above is useful to prevent spurious results. For example, there may be a J. Smith who has published articles in Nuclear Physics and therefore his experience is irrelevant to the J. Smith who provided the local activation time annotations 50.

The training module 70 running on the processing circuitry 60 is configured to compute (block 108) weights for annotations performed by the respective annotation personnel responsively to the local activation time annotation experience level of the respective annotation personnel. For example, a weight is computed for annotations performed by J. Smith and another weight is computed for annotations performed by T. Jones, and so on.

In some embodiments, the training module 70 running on the processing circuitry 60 is configured to compute the weights for the annotations performed by the respective annotation personnel responsively to the respective numbers of search matches (e.g., numbers of the scientific literature publications) for the respective annotation personnel. For example, a weight is computed for annotations performed by J. Smith responsively to the 5 search matches (e.g., 5 publications) found in the database for J. Smith, and another weight is computed for annotations performed by T. Jones responsively to the 40 search matches (e.g., 40 publications) found in the database for T. Jones, and so on. The weights may be computed proportionally. For example, if there are N annotation personnel, and the $j^{th}$ annotation person has $P_j$ publications found in the search, the weight $W_i$ for the $i^{th}$ annotation person is equal to:

$$\frac{P_i}{\sum_{j=1}^{N} P_j}$$

The training module 70 running on the processing circuitry 60 is configured to train (block 110) the artificial neural network 68 to find local activation times of IEGM signals responsively to training data including the IEGM signals 48 (received from the EP laboratory subsystems 11) and the corresponding local activation time annotations 50 weighted according to respective computed weights of the respective annotation personnel who annotated respective local activation time annotations 50. For example, the annotations provided by J. Smith are weighted according to the weight computed for J. Smith, and the annotations provided by T. Jones are weighted according to the weight computed for T. Jones and so on.

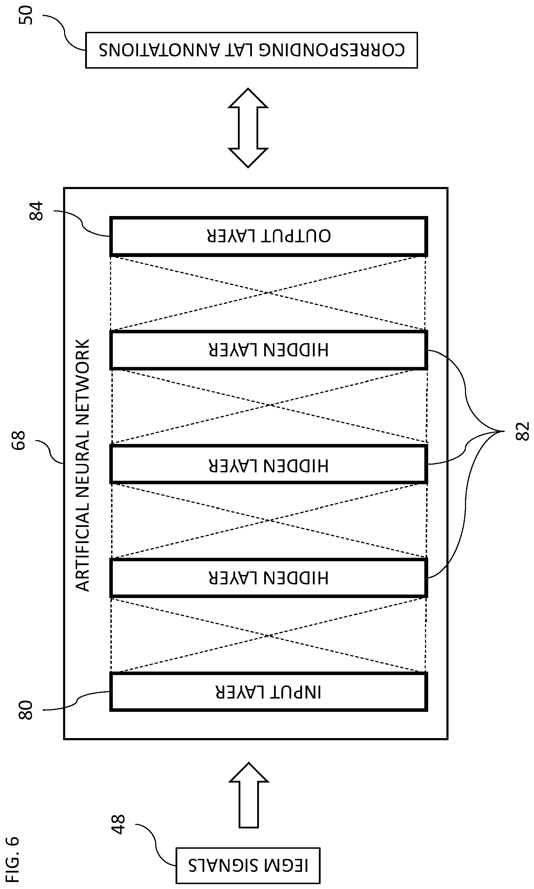

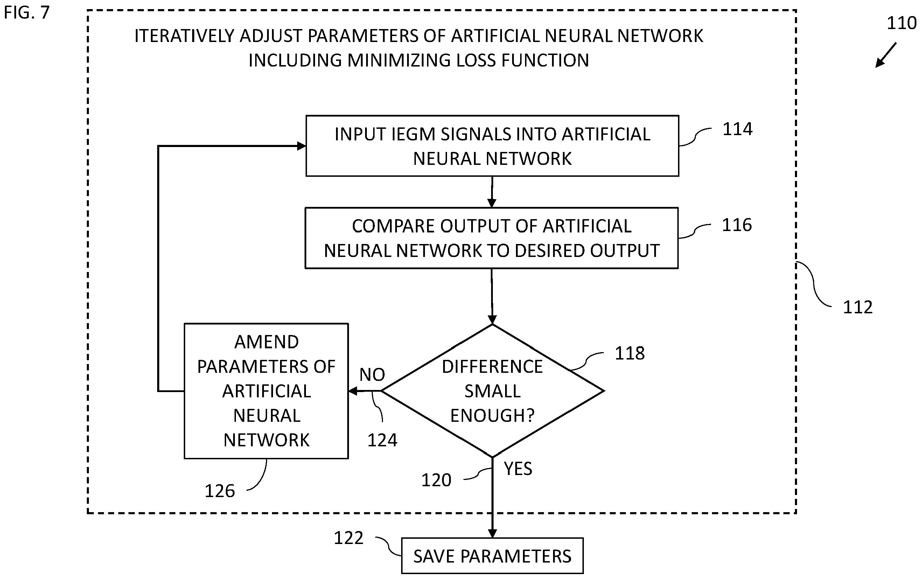

Reference is now made to Figs. 6 and 7. Fig. 6 is a schematic view the artificial neural network 68 for use with the system 10 of Fig. 1. Fig. 7 is a flowchart including sub-steps in the step of block 110 of the method of Fig. 5.

A neural network is a network or circuit of neurons, or in a modern sense, an artificial neural network, composed of artificial neurons or nodes. The connections of the biological neuron are modeled as weights. A positive weight reflects an excitatory connection, while negative values mean inhibitory connections. Inputs are modified by a weight and summed using a linear combination. An activation function may control the amplitude of the output. For example, an acceptable range of output is usually between 0 and 1, or it could be -1 and 1.

These artificial networks may be used for predictive modeling, adaptive control and applications and can be trained via a dataset. Self-learning resulting from experience can occur within networks, which can derive conclusions from a complex and seemingly unrelated set of information.

For completeness, a biological neural network is composed of a group or groups of chemically connected or functionally associated neurons. A single neuron may be connected to many other neurons and the total number of neurons and connections in a network may be extensive. Connections, called synapses, are usually formed from axons to dendrites, though dendrodendritic synapses and other connections are possible. Apart from the electrical signaling, there are other forms of signaling that arise from neurotransmitter diffusion.

Artificial intelligence, cognitive modeling, and neural networks are information processing paradigms inspired by the way biological neural systems process data. Artificial intelligence and cognitive modeling try to simulate some properties of biological neural networks. In the artificial intelligence field, artificial neural networks have been applied successfully to speech recognition, image analysis and adaptive control, in order to construct software agents (in computer and video games) or autonomous robots.

A neural network (NN), in the case of artificial neurons called artificial neural network (ANN) or simulated neural network (SNN), is an interconnected group of natural or artificial neurons that uses a mathematical or computational model for information processing based on a connectionistic approach to computation. In most cases an ANN is an adaptive system that changes its structure based on external or internal information that flows through the network. In more practical terms, neural networks are non-linear statistical data modeling or decision-making tools. They can be used to model complex relationships between inputs and outputs or to find patterns in data.

In some embodiments, the artificial neural network 68 includes a fully connected neural network, e.g., a convolutional neural network. In other embodiments, the artificial neural network 68 may comprise any suitable ANN. The artificial neural network 68 may comprise software executed by the processing circuitry 60 (Fig. 4) and/or hardware modules configured to perform the functions of the artificial neural network 68.

The artificial neural network 68 includes an input layer 80 into which an input is received, and one or more hidden layers 82 which progressively process the input to an output layer 84 from which the output of the artificial neural network 68 is provided. The artificial neural network 68 may include layer weights between the layers 80, 82, 84 of the artificial neural network 68. The artificial neural network 68 manipulates the data received at the input layer 80 according to the values of the various layer weights between the layers 80, 82, 84 of the artificial neural network 68.

The layer weights of the artificial neural network 68 are updated during training of the artificial neural network 68 so that the artificial neural network 68 performs a data manipulation task that the artificial neural network 68 is trained to perform.

The number of layers in the artificial neural network 68 and the width of the layers may be configurable. As the number of layers and width of the layers increases so does the accuracy to which the artificial neural network 68 can manipulate data according to the task at hand. However, a larger number of layers, and wider layers, generally requires more training data, more training time and the training may not converge. By way of example, the input layer 80 may include 400 neurons (e.g., to compress a batch of 400 samples) and the output layer may also include 400 neurons.

Training the artificial neural network 68 is generally an iterative process. One method of training the artificial neural network 68 is now described below. The training module 70 running on the processing circuitry 60 (Fig. 4) is configured to iteratively adjust (block 112) parameters (e.g., layer weights) of the artificial neural network 68 to reduce a difference between an output of the artificial neural network 68 and the local activation time annotations 50 of the IEGM signals 48.

In some embodiments, the training module 70 running on the processing circuitry 60 (Fig. 4) is configured to: minimize a loss function which is a function of the output of the artificial neural network 68 and the local activation time annotations 50 of the IEGM signals 48 weighted according to respective ones of the computed weights (computed in the step of block 108 of Fig. 5); and iteratively adjust the parameters (e.g., layer weights) of the artificial neural network 68 responsively to minimizing the loss function. In some embodiments, the loss function includes a binary cross entropy (BCE) loss function. Pytorch.org provides an example of a suitable BCE loss function.

Sub-steps of the step of block 112 are now described below.

The training module 70 running on the processing circuitry 60 of the processing circuitry 22 (Fig. 4) is configured to input (block 114) the IEGM signals 48 into the input layer 80 of the artificial neural network 68. The training module 70 running on the processing circuitry 60 (Fig. 4) is configured to compare (block 116) the output of the artificial neural network 68 (e.g., the output of the output layer 84) with the desired output, i.e., the corresponding local activation time annotations 50 of the IEGM signals 48, for example, using a suitable loss function which takes into account the weights (computed for the respective annotation personnel) for the respective local activation time annotations 50.

The output of the artificial neural network 68 includes various vectors corresponding with the IEGM signals 48 input into the artificial neural network 68. Each of the output vectors includes components, each component with a floating-point value (for example, between 0 and 1). Each of the desired outputs is expressed as a one-hot vector in which all the components of the vectors have zero values except for one of the components which has a value of one corresponding with the time value of the respective local activation time annotation 50.

For example, if there is a set of vectors A, B, C output by the artificial neural network 68 and a corresponding set of vectors representing corresponding local activation time annotations A', B', and C', the training module 70 of the processing circuitry 60 (Fig. 4) uses the loss function to compare A with A', B with B', C with C' and so on, based on the weights of the annotation personnel who annotated A', B' and C', respectively.

At a decision block 118, the training module 70 running on the processing circuitry 60 (Fig. 4) is configured to determine if the difference between the output of the artificial neural network 68 and desired output is small enough. If the difference between the output of the artificial neural network 68 and the desired output is small enough (branch 120), the training module 70 running on the processing circuitry 60 (Fig. 4) is configured to save (block 122) the parameters (e.g., layer weights) of the artificial neural network 68 for future use.

If the difference is not small enough (branch 124), the training module 70 running on the processing circuitry 60 (Fig. 4) is configured to amend (block 126) parameters (e.g., layer weights) of the artificial neural network 68 to reduce the difference between the output of the artificial neural network 68 and the desired output of the artificial neural network 68 according to the loss function. The difference being minimized in the above example is the overall difference between all the outputs of the artificial neural network 68 and all the desired outputs (e.g., local activation time annotations 50) according to the loss function. The training module 70 running on the processing circuitry 60 (Fig. 4) is configured to amend the parameters using any suitable optimization algorithm, for example, a gradient descent algorithm such as Adam Optimization. The steps of blocks 114-118 are then repeated.

Reference is now made to Fig. 8, which is a flowchart 150 including steps in a method to process an intracardiac electrogram signal using the trained artificial neural network 68 of Fig. 6. Reference is also made to Fig. 4.

The mapping module 72 (or any other suitable module) running on the processing circuitry 60 is configured to receive (block 152) an IEGM signal, from one of the EP laboratory sub-systems 11. The mapping module 72 (or any other suitable module) running on the processing circuitry 60 is configured to apply (block 154) the trained artificial neural network 68 to the received IEGM signal to provide an indication of a local activation time of the received IEGM signal. The output of the artificial neural network 68 may include a vector having components (e.g., 400 components), with each component having a floating value (e.g., a decimal value), for example, between 0 and 1. The floating values represent respective probabilities that the respective vector components are the LAT value for the input IEGM signal. Therefore, the highest floating value is associated with the highest probability and therefore indicates the LAT value that should be used for the input IEGM signal. The mapping module 72 running on the processing circuitry 60 may be configured to receive the output of the artificial neural network 68 and find the highest floating value of the components of the vector output by the artificial neural network 68 and compute the LAT value for the received IEGM signal responsively to the position of the vector component with the highest floating value.

Reference is now made to Fig. 9, which is a schematic view of a displayed electroanatomic map 160 rendered by the system 10 of Fig. 1. Reference is also made to Fig. 8. The mapping module 72 (or any other suitable module) running on the processing circuitry 60 is optionally configured to generate (block 156) the electroanatomic map 160 responsively to the indication of the local activation time provided in the step of block 154 and other similar EP data. In some embodiments the processing circuitry 60 is configured to provide the electroanatomic map 160 to the EP laboratory sub-system 11 which provided the IEGM signals used to generate the electroanatomic map 160. The mapping module 72 is optionally configured to provide (block 158) the LAT found in the step of block 154 to the EP laboratory sub-system 11 which provided the IEGM signal for which the LAT was found.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 72% to 108%.

Various features of the disclosure which are, for clarity, described in the contexts of separate embodiments may also be provided in combination in a single embodiment. Conversely, various features of the invention which are, for brevity, described in the context of a single embodiment may also be provided separately or in any suitable sub-combination.

The embodiments described above are cited by way of example, and the present disclosure is not limited by what has been particularly shown and described hereinabove. Rather the scope of the disclosure includes both combinations and sub-combinations of the various features described hereinabove. The scope of the present invention is nevertheless defined by the appended claims. 2.

**Claims**

1. A system (10) to find local activation times of intracardiac electrogram (IEGM) signals, comprising a remote server (26) including processing circuitry (60) configured to:

   receive (102), from electrophysiological laboratory sub-systems (11), first IEGM signals and corresponding local activation time annotations of the first IEGM signals manually annotated by respective annotation personnel;
   compute (108) weights for annotations performed by respective ones of the annotation personnel responsively to a local activation time annotation experience level of the respective ones of the annotation personnel;
   train (104) an artificial neural network (68) to find local activation times of IEGM signals responsively to the first IEGM signals and the corresponding local activation time annotations weighted according to respective ones of the computed weights of the respective ones of the annotation personnel who annotated respective ones of the local activation time annotations;
   receive (152) a second IEGM signal; and
   apply (154) the trained artificial neural network to the received second IEGM signal to provide an indication of a local activation time of the received second IEGM signal.

2. The system according to claim 1, further comprising the electrophysiological laboratory sub-systems, each electrophysiological laboratory sub-system comprising:

> a catheter configured to be inserted into at least one cardiac chamber of at least one living subject, and to capture respective ones of the first IEGM signals from the at least one cardiac chamber;
> a display; and
> processing circuitry configured to:
>
>> render the respective ones of the first IEGM signals to the display;
>> receive the corresponding ones of the local activation time annotations of the displayed first IEGM signals manually annotated by a respective one of the annotation personnel; and
>> provide the respective ones of the first IEGM signals and the corresponding ones of the local activation time annotations to the remote server.

3. The system according to claim 1, wherein the processing circuitry is configured to:

> search a database of scientific literature publications responsively to respective ones of the annotation personnel yielding respective numbers of search matches indicative of the local activation time annotation experience level of the respective ones of the annotation personnel; and
> compute the weights for the annotations performed by respective ones of the annotation personnel responsively to the respective numbers of search matches for the respective ones of the annotation personnel.

4. The system according to claim 3, wherein the processing circuitry is configured to limit searching of the database to scientific literature publications describing local activation time annotation.

5. The system according to claim 3, wherein the respective numbers of search matches are respective numbers of the scientific literature publications matching respective ones of the annotation personnel.

6. The system according to claim 3, wherein the processing circuitry is configured to:

input the first IEGM signals into the artificial neural network; and
iteratively adjust parameters of the artificial neural network responsively to an output of the artificial neural network and the local activation time annotations of the first IEGM signals.

7. The system according to claim 6, wherein the processing circuitry is configured to:

> minimize a loss function which is a function of the output of the artificial neural network and the local activation time annotations of the first IEGM signals weighted according to respective ones of the computed weights; and
> iteratively adjust the parameters of the artificial neural network responsively to minimizing the loss function.

8. The system according to claim 7, wherein the loss function includes a binary cross entropy loss function.

9. The system according to claim 1, wherein the processing circuitry is configured to generate an electroanatomic map responsively to the indication of the local activation time.

10. A software product, comprising a non-transient computer-readable medium in which program instructions are stored, which instructions, when read by a central processing unit (CPU), cause the CPU to:

> Receive (102), from electrophysiological laboratory sub-systems (11), first IEGM signals and corresponding local activation time annotations of the first IEGM signals manually annotated by respective annotation personnel;
> compute (108) weights for annotations performed by respective ones of the annotation personnel responsively to a local activation time annotation experience level of the respective ones of the annotation personnel
> train (104) an artificial neural network (68) to find local activation times of IEGM signals responsively to the first IEGM signals and the corresponding local activation time annotations weighted according to respective ones of the computed weights of the respective ones of the annotation personnel who annotated respective ones of the local activation time annotations;
> receive (152) a second IEGM signal; and
> apply (154) the trained artificial neural network to the received second IEGM signal to provide an indication of a local activation time of the received second IEGM signal.

**Patentansprüche**

1. System (10) zum Finden lokaler Aktivierungszeiten von intrakardialen Elektrogrammsignalen (IEGM-Signalen), umfassend einen Remote-Server (26), der eine Verarbeitungsschaltung (60) einschließt, die konfiguriert ist zum:

   Empfangen (102), von elektrophysiologischen Laborteilsystemen (11), erster IEGM-Signale und entsprechender Anmerkungen der lokalen Aktivierungszeit der ersten IEGM-Signale, die durch entsprechendes Anmerkungspersonal manuell angemerkt werden;
   Berechnen (108) von Gewichtungen für Anmerkungen, die durch jeweilige des Anmerkungspersonals durchgeführt werden, als Reaktion auf ein Anmerkungserfahrungsniveau der lokalen Aktivierungszeit der jeweiligen des Anmerkungspersonals;
   Trainieren (104) eines künstlichen neuronalen Netzwerks (68), um lokale Aktivierungszeiten von IEGM-Signalen als Reaktion auf die ersten IEGM-Signale und die entsprechenden Anmerkungen der lokalen Aktivierungszeit zu finden, die gemäß jeweiligen der berechneten Gewichtungen der jeweiligen des Anmerkungspersonals gewichtet werden, die die jeweiligen Anmerkungen der lokalen Aktivierungszeit angemerkt haben;
   Empfangen (152) eines zweiten IEGM-Signals; und
   Anwenden (154) des trainierten künstlichen neuronalen Netzwerks auf das empfangene zweite IEGM-Signal, um eine Anzeige einer lokalen Aktivierungszeit des empfangenen zweiten IEGM-Signals bereitzustellen.

2. System nach Anspruch 1, ferner umfassend die elektrophysiologischen Laborteilsysteme, jedes elektrophysiologische Laborteilsystem umfassend:

   einen Katheter, der konfiguriert ist, um in mindestens eine Herzkammer von mindestens einem lebenden Subjekt eingeführt zu werden und um die jeweiligen der ersten IEGM-Signale aus der mindestens einen Herzkammer zu erfassen;
   eine Anzeige; und
   Verarbeitungsschaltlogik, die konfiguriert ist zum:

   Wiedergeben der jeweiligen der ersten IEGM-Signale auf das Display;
   Empfangen der entsprechenden der Anmerkungen der lokalen Aktivierungszeit der angezeigten ersten IEGM-Signale, die durch einen jeweiligen des Anmerkungspersonals manuell angemerkt werden; und
   Bereitstellen der jeweiligen der ersten IEGM-Signale und der entsprechenden der Anmerkungen der lokalen Aktivierungszeit an den Remote-Server.

3. System nach Anspruch 1, wobei die Verarbeitungsschaltung konfiguriert ist zum:

   Durchsuchen einer Datenbank mit Veröffentlichungen wissenschaftlicher Literatur als Reaktion auf jeweilige des Anmerkungspersonals, die jeweilige Zahlen von Suchübereinstimmungen einbringen, die das Anmerkungserfahrungsniveau der lokalen Aktivierungszeit der jeweiligen des Anmerkungspersonals anzeigt; und
   Berechnen der Gewichte für die Anmerkungen, die durch jeweilige des Anmerkungspersonals durchgeführt werden, als Reaktion auf die jeweiligen Zahlen von Suchübereinstimmungen für die jeweiligen des Anmerkungspersonals.

4. System nach Anspruch 3, wobei die Verarbeitungsschaltung konfiguriert ist, um ein Suchen in der Datenbank mit Veröffentlichungen wissenschaftlicher Literatur zu beschränken, die Anmerkungen der lokalen Aktivierungszeiten beschreibt.

5. System nach Anspruch 3, wobei die jeweiligen Zahlen der Suchübereinstimmungen die jeweiligen Zahlen der Veröffentlichungen wissenschaftlicher Literatur sind, die mit den jeweiligen Zahlen des Anmerkungspersonals übereinstimmen.

6. System nach Anspruch 3, wobei die Verarbeitungsschaltung konfiguriert ist zum:

   Eingeben der ersten IEGM-Signale in das künstliche neuronale Netzwerk; und
   Iteratives Anpassen der Parameter des künstlichen neuronalen Netzwerks, als Reaktion auf eine Ausgabe des künstlichen neuronalen Netzwerks und die Anmerkungen der lokalen Aktivierungszeit der ersten IEGM-Signale.

7. System nach Anspruch 6, wobei die Verarbeitungsschaltung konfiguriert ist zum:

   Minimieren einer Verlustfunktion, die eine Funktion der Ausgabe des künstlichen neuronalen Netzwerks und der Anmerkungen der lokalen Aktivierungszeit der ersten IEGM-Signale ist, die gemäß der jeweiligen der berechneten Gewichtungen gewichtet werden; und
   Iteratives Anpassen der Parameter des künstlichen neuronalen Netzwerks als Reaktion auf das Minimieren der Verlustfunktion.

**8.** System nach Anspruch 7, wobei die Verlustfunktion eine binäre Kreuzentropieverlustfunktion einschließt.

**9.** System nach Anspruch 1, wobei die Verarbeitungsschaltung konfiguriert ist, um, als Reaktion auf die Anzeige der lokalen Aktivierungszeit, eine elektroanatomische Karte zu erzeugen.

**10.** Softwareprodukt, umfassend ein nicht flüchtiges computerlesbares Medium, in dem Programmanweisungen gespeichert sind, wobei die Anweisungen, wenn sie durch eine zentrale Verarbeitungseinheit (CPU) gelesen werden, die CPU veranlassen zum:

Empfangen (102) von elektrophysiologischen Laborteilsystemen (11), erster IEGM-Signale und entsprechender Anmerkungen der lokalen Aktivierungszeit der ersten IEGM-Signale, die durch entsprechendes Anmerkungspersonal manuell angemerkt werden;

Berechnen (108) von Gewichtungen für Anmerkungen, die durch jeweilige des Anmerkungspersonals durchgeführt werden, als Reaktion auf ein Anmerkungserfahrungsniveau der lokalen Aktivierungszeit des jeweiligen des Anmerkungspersonals

Trainieren (104) eines künstlichen neuronalen Netzwerks (68), um lokale Aktivierungszeiten von IEGM-Signalen als Reaktion auf die ersten IEGM-Signale und die entsprechenden Anmerkungen der lokalen Aktivierungszeit zu finden, die gemäß jeweiligen der berechneten Gewichtungen der jeweiligen des Anmerkungspersonals gewichtet werden, die die jeweiligen Anmerkungen der lokalen Aktivierungszeit angemerkt haben;

Empfangen (152) eines zweiten IEGM-Signals; und

Anwenden (154) des trainierten künstlichen neuronalen Netzwerks auf das empfangene zweite IEGM-Signal, um eine Anzeige einer lokalen Aktivierungszeit des empfangenen zweiten IEGM-Signals bereitzustellen.

**Revendications**

**1.** Système (10) permettant de trouver des temps d'activation locaux de signaux d'électrocardiogrammes endocavitaires (IEGM), comprenant un serveur à distance (26) comportant une circuiterie de traitement (60) configurée pour :

recevoir (102), à partir des sous-systèmes de laboratoire électrophysiologique (11), des premiers signaux IEGM et des annotations correspondantes de temps d'activation local des premiers signaux IEGM, annotés manuellement par un personnel d'annotation respectif ;

calculer (108) des poids pour des annotations effectuées par le personnel respectif du personnel d'annotation en fonction d'un niveau d'expérience d'annotation de temps d'activation local du personnel respectif du personnel d'annotation ;

entraîner (104) un réseau neuronal artificiel (68) pour trouver les temps d'activation locaux des signaux IEGM en fonction des premiers signaux IEGM et des annotations correspondantes de temps d'activation local, pondérés selon des poids respectifs des poids calculés par le personnel respectif du personnel d'annotation qui a annoté les annotations respectives des annotations de temps d'activation local ;

recevoir (152) un second signal IEGM ; et

appliquer (154) le réseau neuronal artificiel entraîné au second signal IEGM reçu afin de fournir une indication d'un temps d'activation local du second signal IEGM reçu.

**2.** Système selon la revendication 1, comprenant en outre les sous-systèmes de laboratoire électrophysiologique,

chaque sous-système de laboratoire électrophysiologique comprenant :

un cathéter conçu pour être inséré dans au moins une chambre cardiaque d'au moins un sujet vivant, et pour capturer des signaux respectifs des premiers signaux IEGM provenant de l'au moins une chambre cardiaque ;

un dispositif d'affichage ; et

une circuiterie de traitement configurée pour :

rendre les signaux respectifs des premiers signaux IEGM sur le dispositif d'affichage ;

recevoir les annotations correspondantes des annotations de temps d'activation local des premiers signaux IEGM affichés, annotées manuellement par un personnel respectif du personnel d'annotation ; et

fournir les signaux respectifs des premiers signaux IEGM et les annotations correspondantes des annotations de temps d'activation local au serveur à distance.

**3.** Système selon la revendication 1, dans lequel la circuiterie de traitement est configurée pour :

effectuer des recherches dans une base de données de publications de littérature scientifique en fonction du personnel respectif du personnel d'annotation produisant des nombres respectifs de correspondances de recherche indiquant le niveau d'expérience d'annotation de temps d'activation local du personnel respectif du personnel d'annotation ; et

calculer les poids pour les annotations effectuées par le personnel respectif du personnel d'annotation en fonction des nombres respectifs de correspondances de recherche pour le personnel respectif du personnel d'annotation.

**4.** Système selon la revendication 3, dans lequel la circuiterie de traitement est configurée pour limiter la recherche dans la base de données aux publications de littérature scientifique décrivant une annotation de temps d'activation local.

**5.** Système selon la revendication 3, dans lequel les nombres respectifs de correspondances de recherche sont les nombres respectifs des publications de littérature scientifique correspondant au personnel respectif du personnel d'annotation.

**6.** Système selon la revendication 3, dans lequel la circuiterie de traitement est configurée pour :

introduire les premiers signaux IEGM dans le réseau neuronal artificiel ; et
ajuster itérativement des paramètres du réseau neuronal artificiel en fonction d'une sortie du réseau neuronal artificiel et des annotations de temps d'activation local des premiers signaux IEGM.

**7.** Système selon la revendication 6, dans lequel la circuiterie de traitement est configurée pour :

minimiser une fonction de perte qui est une fonction de la sortie du réseau neuronal artificiel et des annotations de temps d'activation local des premiers signaux IEGM pondérés selon des poids respectifs des poids calculés ; et
ajuster itérativement les paramètres du réseau neuronal artificiel en fonction de la minimisation de la fonction de perte.

**8.** Système selon la revendication 7, dans lequel la fonction de perte comporte une fonction de perte d'entropie croisée binaire.

**9.** Système selon la revendication 1, dans lequel la circuiterie de traitement est configurée pour générer une carte électroanatomique en fonction de l'indication du temps d'activation local.

**10.** Produit logiciel, comprenant un support non transitoire lisible par ordinateur dans lequel sont stockées des instructions de programme, lesquelles instructions, lorsqu'elles sont lues par une unité centrale de traitement (UCT), amènent l'UCT à :

recevoir (102), à partir des sous-systèmes de laboratoire électrophysiologique (11), des premiers signaux IEGM et des annotations correspondantes de temps d'activation local des premiers signaux IEGM, annotés manuellement par le personnel d'annotation respectif ;
calculer (108) des poids pour des annotations effectuées par le personnel respectif du personnel d'annotation en fonction du niveau d'expérience d'annotations de temps d'activation local du personnel respectif du personnel d'annotation
entraîner (104) un réseau neuronal artificiel (68) pour trouver les temps d'activation locaux des signaux IEGM en fonction des premiers signaux IEGM et des annotations correspondantes de temps d'activation local, pondérés selon des poids respectifs des poids calculés par le personnel respectif du personnel d'annotation qui a annoté les annotations respectives des annotations de temps d'activation local ;
recevoir (152) un second signal IEGM ; et
appliquer (154) le réseau neuronal artificiel entraîné au second signal IEGM reçu afin de fournir une indication d'un temps d'activation local du second signal IEGM reçu.

FIG. 1

EP LABORATORY
SUB-SYSTEM

REMOTE SERVER

EP 3 967 218 B1

EP 3 967 218 B1

40

42 — RENDER IEGM SIGNALS TO DISPLAY

44 — RECEIVE LAT ANNOTATIONS (CORRECTIONS)

46 — PROVIDE IEGM SIGNALS AND LAT
ANNOTATIONS TO REMOTE SERVER

FIG. 2

48

50

50

48

29

50

48

FIG. 3

FIG. 4

REMOTE SERVER

MEMORY

PROCESSING CIRCUITRY

MAPPING MODULE

ARTIFICIAL NEURAL NETWORK

TRAINING MODULE

DATA BUS

NETWORK INTERFACE

EP LAB SUB-SYSTEM

EP LAB SUB-SYSTEM

EP LAB SUB-SYSTEM

EP LAB SUB-SYSTEM

10

60

26

68

72

62

70

64

66

27

11

FIG. 5

RECEIVE IEGM SIGNALS AND LAT ANNOTATIONS — 102

100

TRAIN ARTIFICIAL NEURAL NETWORK — 104

SEARCH DATABASE OF SCIENTIFIC LITERATURE PUBLICATIONS PUBLISHED BY ANNOTATION PERSONNEL (LIMITED BY LAT ANNOTATION) — 106

COMPUTE WEIGHTS ACCORDING TO NUMBERS OF SEARCH MATCHES — 108

TRAIN ACCORDING TO IEGM SIGNALS AND LAT ANNOTATIONS WEIGHTED ACCORDING TO COMPUTED WEIGHTS — 110

IEGM SIGNALS — 48

ARTIFICIAL NEURAL NETWORK — 68

| INPUT LAYER — 80 |
| HIDDEN LAYER |
| HIDDEN LAYER |
| HIDDEN LAYER |
| OUTPUT LAYER — 84 |

82

CORRESPONDING LAT ANNOTATIONS — 50

FIG. 6

FIG. 7

110

112 — ITERATIVELY ADJUST PARAMETERS OF ARTIFICIAL NEURAL NETWORK INCLUDING MINIMIZING LOSS FUNCTION

114 — INPUT IEGM SIGNALS INTO ARTIFICIAL NEURAL NETWORK

116 — COMPARE OUTPUT OF ARTIFICIAL NEURAL NETWORK TO DESIRED OUTPUT

118 — DIFFERENCE SMALL ENOUGH?

120 — YES

122 — SAVE PARAMETERS

124 — NO

126 — AMEND PARAMETERS OF ARTIFICIAL NEURAL NETWORK

FIG. 8

152 ┌─────────────────────────┐
    │ RECEIVE IEGM SIGNAL     │                          150
    └─────────────────────────┘

154 ┌───────────────────────────────────────────────────────┐
    │ APPLY TRAINED ARTIFICAL NETWORK TO RECEIVED IEGM SIGNAL │
    └───────────────────────────────────────────────────────┘

156 ┌───────────────────────────────────────────────────────────────┐
    │ GENERATE ELECTROANATOMIC MAP AND PROVIDE TO EP LAB SUB-SYSTEM  │
    └───────────────────────────────────────────────────────────────┘

158 ┌───────────────────────────────────────┐
    │ PROVIDE LAT TO EP LAB SUB-SYSTEM      │
    └───────────────────────────────────────┘

FIG. 9

160

29

EP 3 967 218 B1

**REFERENCES CITED IN THE DESCRIPTION**

**Patent documents cited in the description**

- US 20130123652
- US 20180296108 A1
- US 6226542 B
- US 6301496 B
- US 6892091 B
- US 7536218 B
- US 6814733 B
- US 6997924 B
- US 7156816 B
- US 7756576 B